(19) Europäisches Patentamt
European Patent Office
Office européen des brevets

(11) Veröffentlichungsnummer : **0 414 065 B1**

(12) **EUROPÄISCHE PATENTSCHRIFT**

(45) Veröffentlichungstag der Patentschrift : **19.01.94 Patentblatt 94/03**

(51) Int. Cl.$^5$ : **C07C 45/41**, C07C 47/54, C07C 47/542, C07C 47/575, C07C 47/02, B01J 23/00

(21) Anmeldenummer : **90115371.8**

(22) Anmeldetag : **10.08.90**

(54) **Verfahren zur Herstellung von Aldehyden.**

(30) Priorität : **23.08.89 DE 3927786**

(43) Veröffentlichungstag der Anmeldung : **27.02.91 Patentblatt 91/09**

(45) Bekanntmachung des Hinweises auf die Patenterteilung : **19.01.94 Patentblatt 94/03**

(84) Benannte Vertragsstaaten : **BE DE ES FR GB IT NL**

(56) Entgegenhaltungen : **EP-A- 0 290 096**

(73) Patentinhaber : **BAYER AG**
**D-51368 Leverkusen (DE)**

(72) Erfinder : **Joentgen, Winfried, Dr.**
**Merkenicher Ring Strasse 149**
**D-5000 Köln 71 (DE)**
Erfinder : **Fiege, Helmut, Dr.**
**Walter-Flex-Strasse 23**
**D-5090 Leverkusen 1 (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 414 065 B1

## Beschreibung

Die vorliegende Erfindung betrifft ein Verfahren zur Herstellung von aromatischen und aliphatischen Aldehyden durch katalytische Gasphasenhydrierung aromatischer bzw. aliphatischer Carbonsäuren oder einiger ihrer Derivate mit Hilfe eines auf Titandioxid oder Vanadium(IV)oxid basierenden Katalysatorsystems.

Es ist bekannt, daß man aromatische und aliphatische Carbonsäuren oder Carbonsäureester mit molekularem Wasserstoff unter Verwendung verschiedener Katalysatoren zu den korrespondierenden Aldehyden reduzieren kann. So können nach US 3.935.265 Alkylester aromatischer Carbonsäuren bei 400 - 600°C an $Al_2O_3$ mit Wasserstoff reduziert werden; beispielsweise wird Benzoesäuremethylester mit einer Selektivität von 37 % zu Benzaldehyd umgesetzt, wobei der Umsatz des Esters 39 % beträgt. Es ist ferner bekannt, daß Zirkondioxid allein (US 4.328.373) oder in Kombination mit den Oxiden anderer Metalle wie Chrom, Mangan, Eisen, Zink, Kobalt, Wismut, Blei, Rhenium oder Elementen der III. Hauptgruppe wie Bor, Aluminium, Gallium, Indium oder Thallium (EP 150 961) oder gemeinsam mit Oxiden von Elementen der Lanthanid-Gruppe (US 4.328.373) in der Lage ist, Carbonsäuren oder deren Ester mit Wasserstoff zu den entsprechenden Aldehyden zu reduzieren. Die genannte US 4.328.373 offenbart weiter, daß ähnlich wie Zirkondioxid auch die Oxide des Yttriums, Cers, Praseodyms, Thoriums und Urans wirken, wobei diese wie Zirkondioxid auch in Kombination mit Aluminiumoxid eingesetzt werden können.

Auch Mangandioxid ist nach US 4.585.899 in Verbindung mit Aluminiumoxid und Siliciumdioxid wirksam. Nach EP 290 096 kann Mangandioxid auf verschiedene Träger, wie Aluminiumoxid, Zirkondioxid, Titandioxid, Cer(III)oxid, Hafniumdioxid oder Niob(V)oxid aufgebracht werden. Eine spezifische Wirkung der Träger in Kombination mit dem Mangandioxid wird hier nicht erwähnt. Entscheidend für die Aktivität des Katalysators ist nach EP 290 096 die Art der Herstellung des Manganoxids.

Die Wirksamkeit einer Kombination Mangandioxid/Titandioxid wird in EP 290 096 nicht beschrieben. Nach eigenen Versuchen zeigte ein beispielsweise aus 23 Gew.-% Mangandioxid auf Titandioxid bestehender und durch gemeinsame Fällung der Komponenten hergestellter Katalysator bei 370°C für die oben genannte Aufgabe keine besondere Wirksamkeit. Bei höherer Temperatur, insbesondere oberhalb von 400°C, stieg zwar die Aktivität der Umwandlung von Carbonsäuren oder deren Derivaten, sank jedoch die Selektivität zu den angestrebten Aldehyden in einem Maße, das technisch nicht vertretbar ist.

Überraschend wurde nun gefunden, daß Katalysatorsysteme aus Titandioxid und/oder Vanadiumoxid und einem oder mehreren Oxiden spezieller Co-Metalle hohe Umwandlungsraten bei hoher Selektivität zu den gewünschten Aldehyden ergeben.

Die Erfindung betrifft daher ein Verfahren zur Herstellung von aromatischen und aliphatischen Aldehyden durch katalytische Gasphasenhydrierung der korrespondierenden aromatischen bzw. aliphatischen Carbonsäuren oder ihrer Ester, Anhydride oder Halogenide bei höherer Temperatur mit Wasserstoff, das dadurch gekennzeichnet ist, daß man ein Katalysatorsystem aus Oxiden des Titans und/oder des Vanadiums, wobei mindestens ein Teil des Vanadiums in der Oxidationsstufe IV vorliegt, und eines oder mehrerer Co-Metalle einsetzt, wobei die Co-Metalle aus der Gruppe Chrom, Molybdän, Kobalt, Nickel, Zink, Cadmium und Kupfer ausgewählt werden.

In bevorzugter Weise wird (werden) das (die) Co-Metall(e) aus der Gruppe Chrom, Molybdän, Nickel, Zink, Cadmium und Kupfer, besonders bevorzugt aus der Gruppe Chrom, Molybdän, Zink, Cadmium und Kupfer ausgewählt. In vielen Fällen wurden günstige Ergebnisse besonders mit der Kombination Titandioxid/Chrom(III)oxid oder Vanadium(IV)oxid/Chrom(III)oxid erhalten.

Das erfindungsgemäß einzusetzende Katalysatorsystem enthält 1 bis 25 g-Äquivalente, vorzugsweise 3 bis 12 g-Äquivalente des (der) gewünschten Co-Metall(e), bezogen auf 100 g-Äquivalente aller vorhandenen Metalle.

Wenngleich neben dem Titandioxid und/oder Vanadiumoxid die Oxide von zwei oder mehr Co-Metallen im erfindungsgemäß einzusetzenden Katalysatorsystem vorliegen können, ist es bevorzugt, nur das Oxid eines Co-Metalls einzusetzen.

Die erfindungsgemäß einzusetzenden Katalysatorsysteme können aus leicht zugänglichen Materialien nach einfachen Verfahren hergestellt werden. Die Verwendung teurer Ausgangsstoffe wie Zirkondioxid oder Seltenerdenoxiden wird vermieden. Dennoch zeigen diese erfindungsgemäß einzusetzenden Katalysatorsysteme bei ähnlichen Selektivitäten zu den gewünschten Aldehyden teilweise noch höhere Aktivitäten bezüglich der Umsetzungsrate als die aus der Literatur bekannten Katalysatorsysteme.

Die Herstellung der erfindungsgemäß einsetzbaren Katalysatorsysteme kann beispielsweise durch Imprägnieren von Titandioxid mit einer geeigneten Metallsalzlösung eines oder mehrerer Co-Metalle oder durch gemeinsame Fällung der Metallkomponenten sowie anschließende Trocknung und Kalzinierung erfolgen.

Im ersteren Fall können vorgefertigte Pellets aus Titandioxid durch wiederholtes Sprühen oder Tränken mit einer Lösung von bei höherer Temperatur zersetzlichen Salzen eines oder mehrerer Co-Metalle imprägniert

werden. Die imprägnierten Pellets werden getrocknet und bei 400 bis 800°C, bevorzugt bei 500 bis 750°C, kalziniert.

Im letzteren Fall kann ein wasserlösliches Titansalz und ein oder mehrere Metallsalze des oder der Co-Metalle beispielsweise bei einem pH-Wert von 7 bis 9 mit wäßrigem Ammoniak oder mit Natriumhydroxid oder einem anderen geeigneten basischen Fällungsmittel gefällt werden. Die gefällten Hydroxide werden anschließend gewaschen und beispielsweise 24 Stunden bei 100 bis 150°C, gegebenenfalls unter Anlegung von Vakuum, getrocknet und anschließend ebenfalls bei 400 bis 800°C, bevorzugt bei 500 bis 750°C, kalziniert. Als Zeit für das Kalzinieren kommen 4 bis 8 Stunden in Frage. Eine Abwandlung dieser Herstellungsmethode besteht darin, zu einer gekühlten Metallsalzlösung des oder der Co-Metalle Titantetrachlorid zu geben. Die weitere Verarbeitung (Fällung, Trocknung und Kalzinierung) erfolgt in der bereits beschriebenen Weise.

Das nach dem Kalzinieren erhaltene Katalysatorsystem kann nach Zerkleinerung auf die gewünschte Teilchengröße direkt eingesetzt werden. Das sehr fein gemahlene Katalysatorsystem kann jedoch auch zu Tabletten gepreßt werden; hierbei kann ein Tablettierhilfsmittel zur Verbesserung der Gleiteigenschaften (beispielsweise Graphit) mitverwendet werden.

Von den beschriebenen Herstellungsvarianten hat sich die unter Verwendung von Titantetrachlorid als besonders günstig herausgestellt.

Die Herstellung des erfindungsgemäß einzusetzenden Katalysatorsystems auf der Basis von Vanadiumoxid kann ebenfalls nach den oben näher beschriebenen Herstellungsmethoden erfolgen. Anstelle der Titankomponente wird hier jedoch die entsprechende Vanadiumverbindung eingesetzt. Als Vanadiumverbindung können hierbei Vanadium-IV-oxid, Gemische aus Vanadium-III- und Vanadium-IV-oxid sowie wasserlösliche Vanadium-IV-verbindungen, wie Vanadylsulfat oder Vanadyloxalat, verwendet werden. Als besonders günstig hat sich eine Herstellungsvariante unter Einsatz vom Vanadylsulfat, das durch Reduktion von Vanadium-V-oxid mit Schwefeldioxid in schwefelsaurer Lösung sehr leicht zugänglich ist, oder von Vanadyloxalat, das durch entsprechende Reduktion mit Oxalsäure zugänglich ist, erwiesen.

Nach Zusammengeben der Vanadylverbindung und einem oder mehreren wasserlöslichen Salz(en) eines oder mehrerer Co-Metalle werden die Metalle bei pH 7-9 als Hydroxide gefällt. Die Hydroxide werden gewaschen, getrocknet und bei 350-700°C, bevorzugt 400-600°C kalziniert.

Mit Hilfe des erfindungsgemäß einzusetzenden Katalysatorsystems können eine Vielzahl von aromatischen und aliphatischen Carbonsäuren, Carbonsäureestern, Carbonsäureanhydriden und Carbonsäurehalogeniden in die zugehörigen Aldehyde übergeführt werden. Die aliphatischen Carbonsäuren beziehungsweise ihre Derivate schließen die araliphatischen und die cycloaliphatischen Carbonsäuren und ihre Derivate ein.

Als aromatische und aliphatische Carbonsäuren und Derivate seien insbesondere solche der Formeln

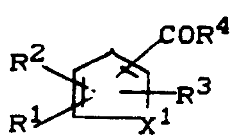 (I) und 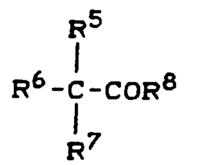 (II)

genannt, in denen

- $R^1$ Wasserstoff, geradkettiges oder verzweigtes $C_1$-$C_8$-Alkyl, geradkettiges oder verzweigtes $C_1$-$C_8$-Alkoxy, $R^3$-substituiertes Phenyl, Naphthyl, $R^3$-substituiertes Phenoxy, $R^3$-substituiertes Benzyl, $R^3$-substituiertes Benzyloxy, Hydroxy, Amino, NH-($C_1$-$C_8$-alkyl), N-($C_1$-$C_8$-alkyl)$_2$, Halogen oder $COR^4$ ist,
- $R^2$ für Wasserstoff, geradkettiges oder verzweigtes $C_1$-$C_8$-Alkyl, geradkettiges oder verzweigtes $C_1$-$C_8$-Alkoxy oder $R^3$-substituiertes Phenyl steht,
  wobei $R^1$ und $R^2$ gemeinsam einen kondensierten Benzolring bilden können, der mit Hydroxy, Amino, Methyl, Ethyl, Methoxy oder Ethoxy substituiert sein kann,
- $R^3$ Wasserstoff, geradkettiges oder verzweigtes $C_1$-$C_8$-Alkyl, geradkettiges oder verzweigtes $C_1$-$C_8$-Alkoxy, Hydroxy, Amino oder Halogen sein kann,
- $R^4$ Hydroxy, $C_1$-$C_4$-alkoxy, Chlor, Brom oder

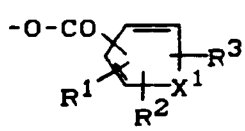

bedeutet,
wobei im letzteren Falle (Anhydridbildung) $R^1$ nicht die Bedeutung $COR^4$ annimmt,

$X^1$ für -O-, -N-, -S-, -N=CH- oder -CH=CH- steht,

$R^5$ Wasserstoff oder geradkettiges oder verzweigtes $C_1$-$C_8$-Alkyl bedeutet,

$R^6$ für geradkettiges oder verzweigtes $C_1$-$C_8$-Alkyl, $R^3$-substituiertes Phenyl oder Halogen steht,

$R^7$ für geradkettiges oder verzweigtes $C_1$-$C_8$-Alkyl oder $R^3$-substituiertes Phenyl steht,
wobei das $C_1$-$C_8$-Alkyl durch Halogen, Methoxy oder Ethoxy substituiert sein kann und wobei weiterhin $R^6$ und $R^7$ gemeinsam Dimethylen, Tetramethylen oder Pentamethylen bedeuten, und

$R^8$ Hydroxy, $C_1$-$C_4$-Alkoxy, Chlor, Brom oder die Gruppe -O-CO-C($R^5$,$R^6$,$R^7$) bedeutet.

Als geradkettiges oder verzweigtes $C_1$-$C_8$-Alkyl sei beispielsweise Methyl, Ethyl, Propyl, Isopropyl, Butyl, Isobutyl, tert.-Butyl, die isomeren Pentyle, Hexyle oder Octyle genannt. In bevorzugter Weise seien die genannten $C_1$-$C_4$-Alkylreste genannt, besonders bevorzugt Methyl oder Ethyl.

Als geradkettiges oder verzweigtes $C_1$-$C_8$-Alkoxy sei beispielsweise Methoxy, Ethoxy, Propoxy, Isopropoxy, Butoxy, Isobutoxy, tert.-Butoxy, die isomeren Pentyloxy, Hexyloxy und Octyloxy genannt. Bevorzugtes Alkoxy hat 1 bis 4 C-Atome und ist besonders bevorzugt Methoxy oder Ethoxy.

Als Halogen sei Fluor, Chlor, Brom, bevorzugt Fluor oder Chlor genannt.

$R^1$ und $R^2$ können gemeinsam einen kondensierten Benzolring bilden, so daß die aromatische Carbonsäure oder deren Derivat vom System des Naphthalins, des Chinolins, des Isochinolins, des Indols, des Cumarons oder des Thionaphthens stammt.

Als Carbonsäure oder deren Derivat seien die Carbonsäure selbst, ihre Ester mit einem $C_1$-$C_4$-Alkohol, ihre Chloride, Bromide oder ihre Anhydride genannt.

Für den Fall, daß $R^1$ den Rest $COR^4$ bedeutet, handelt es sich um eine Dicarbonsäure oder ihr Derivat, woraus der entsprechende Dialdehyd erhalten wird.

Das geradkettige oder verzweigte $C_1$-$C_8$-Alkyl kann insbesondere in den aliphatischen Carbonsäuren oder deren Derivaten durch Halogen wie Fluor, Chlor, Brom, bevorzugt Fluor oder Chlor, durch Methoxy oder Ethoxy substituiert sein.

Für den Fall, daß bei den aliphatischen Carbonsäuren oder Derivaten ein oder mehrere Reste Phenyl bedeuten, gelangt man in die Reihe der araliphatischen Carbonsäuren.

Weiterhin können $R^6$ und $R^7$ gemeinsam Dimethylen, Tetramethylen oder Pentamethylen bedeuten, so daß man in die Reihe der cycloaliphatischen Carbonsäuren mit einem 3-, 5- oder 6-Ring gelangt. Bevorzugt können $R^6$ und $R^7$ gemeinsam Tetramethylen oder Pentamethylen bedeuten.

Unter den aromatischen Carbonsäuren und Derivaten sind solche der Formel

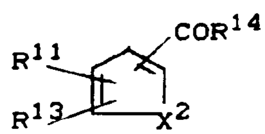

(III)

bevorzugt, in der

$R^{11}$ Wasserstoff, geradkettiges oder verzweigtes $C_1$-$C_8$-Alkyl, geradkettiges oder verzweigtes $C_1$-$C_8$-Alkoxy, $R^{13}$-substituiertes Phenyl, $R^{13}$-substituiertes Phenoxy, Hydroxy, Amino, NH-($C_1$-$C_8$-alkyl), N-($C_1$-$C_8$-alkyl)$_2$ oder Halogen bedeutet,

$R^{13}$ für Wasserstoff, geradkettiges oder verzweigtes $C_1$-$C_8$-Alkyl, geradkettiges oder verzweigtes $C_1$-$C_8$-Alkoxy, Hydroxy oder Halogen steht,

$R^{14}$ Hydroxy, Methoxy, Ethoxy oder Chlor bedeutet und

$X^2$ für -CH=CH- oder -N=CH-, bevorzugt für -CH=CH- steht.

Unter den aromatischen Carbonsäuren oder deren Derivaten sind solche der Formel

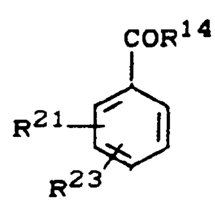

(IV)

besonders bevorzugt, in der

$R^{21}$ Wasserstoff, geradkettiges oder verzweigtes $C_1$-$C_4$-Alkyl, geradkettiges oder verzweigtes $C_1$-$C_4$-Alkoxy, $R^{23}$-substituiertes Phenyl, $R^{23}$-substituiertes Phenoxy, Hydroxy, Amino, Methylamino, Ethylamino, Dimethylamino, Diethylamino, Fluor oder Chlor ist,

$R^{23}$ für Wasserstoff, geradkettiges oder verzweigtes $C_1$-$C_4$-Alkyl, geradkettiges oder verzweigtes $C_1$-$C_4$-Alkoxy, Hydroxy, Fluor oder Chlor steht, und

$R^{14}$ den oben genannten Bedeutungsumfang hat.

Unter den aliphatischen Carbonsäuren und deren Derivaten sind solche der Formel

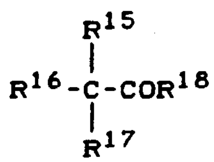

(V)

bevorzugt, in der

$R^{15}$ für Wasserstoff, Methyl oder Ethyl steht,

$R^{16}$ geradkettiges oder verzweigtes $C_1$-$C_8$-Alkyl oder Phenyl bedeutet,

$R^{17}$ Methyl oder Ethyl bedeutet,
wobei weiterhin $R^{16}$ und $R^{17}$ gemeinsam Tetramethylen oder Pentamethylen bedeuten können, und

$R^{18}$ für Hydroxy, Methoxy, Ethoxy oder Chlor steht.

Besonders bevorzugte aromatische und aliphatische Carbonsäuren und deren Derivate sind Benzoesäure, Benzoesäure-methylester, tert.-Butyl-benzoesäure, Phenoxy-benzoesäure, Pivalinsäure, Toluylsäure und m-Chlorbenzoesäure.

Die Gasphasenhydrierung wird in kontinuierlicher Fahrweise durchgeführt, wobei man bei einer Temperatur von 300 bis 500°C, bevorzugt 325 bis 425°C, und einem Druck von 0,1 bis 10 bar, bevorzugt unter atmosphärischem Druck, arbeitet. Die Hydrierung wird mit molekularem Wasserstoff durchgeführt, dem ein Inertgas wie Argon oder Stickstoff zur Verdünnung zugesetzt werden kann. In gleicher Weise kann technischer Wasserstoff eingesetzt werden. Das molare Verhältnis der Carbonsäure oder ihres Derivats zu Wasserstoff liegt bei 1 : 1 - 500, bevorzugt 1 : 5 - 50. Sofern als Carbonsäurederivat ein Anhydrid eingesetzt wird, wird dies wie 2 Mol der Carbonsäure gerechnet.

Die aromatischen und aliphatischen Carbonsäuren bzw. ihre Derivate können als Feststoff, als Schmelze oder als Lösung in einem geeigneten Lösungsmittel, wie Toluol, Benzol, Cyclohexan oder ähnlichen dem Fachmann bekannten, einem Verdampfer zugeführt werden, und danach in dampfförmiger Form dem erfindungsgemäß einzusetzenden Katalysatorsystem zugeführt werden. Die Zufuhrgeschwindigkeit für die Carbonsäure oder deren Derivate beträgt 0,05 bis 2 $h^{-1}$ (LHSV = liquid hourly space velocity); die Zufuhrgeschwindigkeit des Wasserstoffs liegt bei 170 bis 3350 $h^{-1}$ (GHSV = gaseous hourly space velocity).

Die erfindungsgemäß herstellbaren Aldehyde können als Aromastoffe oder als Vorstufen für Wirkstoffe im Pflanzenschutz- oder Pharmabereich eingesetzt werden.

## Beispiele 1 - 8

### Herstellung der Katalysatoren

Zu einer eisgekühlten wäßrigen Lösung von x Mol-% des Co-Metallsalzes tropfte man y Mol-% $TiCl_4$ zu. Die salzsaure Mischung wurde mit wässrigem Ammoniak (bei Cr, Zn, Mo, Mn) oder Natriumhydroxid- bzw. Natriumcarbonat-Lösung (bei Cu, Cd) auf pH 7 bis 9 gebracht. Die ausgefallenen Hydroxide wurden abgesaugt, gewaschen und im Vakuum bei 130°C getrocknet. Anschließend wurden sie bei 700°C (Cr, Mn, Zn, Cu) bzw. 550 bis 600°C (Cd, Mo) calciniert. Nach Zerkleinerung auf Teilchengrößen von 8 bis 18 mesh (1,00 bis 2,5 mm Durchm.) wurde der Katalysator für die Hydrierung eingesetzt. Im Fall von Beispiel 8 wurde $AlCl_3$ anstelle von $TiCl_4$ benutzt.

Mit diesen Katalysatoren wurde die Hydrierung von Benzoesäure unter folgenden Bedingungen durchgeführt:

| | |
|---|---|
| Katalysator: | 25 g (8 - 18 mesh) |
| Temperatur: | 350°C |
| Versuchsdauer: | 4 Stunden |
| Molverhältnis Säure/Wasserstoff: | 1 : 30 |
| Druck: | 1 atm |

Zufuhrrate Säure: 0,09 LHSV ($h^{-1}$)
$H_2$: 550 - 620 GHSV ($h^{-1}$)

| Beispiel | Katalysator Zusammensetzung y Mol-% $TiO_2$ | x Mol-% Me | Umsatz % | Selektivität Aldehyd % | Raum-Zeit-Ausbeute Mol.kg Kat.$^{-1}$.h$^{-1}$ |
|---|---|---|---|---|---|
| 1 | 90 | 10 (Cr) | 79 | 95 | 0,602 |
| 2 | 95 | 5 (Cd) | 91 | 86 | 0,627 |
| 3 | 90 | 10 (Zn) | 23 | 92 | 0,170 |
| 4 | 95 | 5 (Mo) | 81 | 48 | 0,310 |
| 5 | 95 | 5 (Cu) | 72 | 56 | 0,320 |
| 6* | 95 | 5 (Mn) | 1,5 | 42 | 0,005 |
| 7* | 80 | 20 (Mn) | 4 | 70 | 0,020 |
| 8* | 95 (Al) | 5 (Cr) | 4 | 88 | 0,027 |

*) Vergleich

Beispiel 9 und 10

Die Durchführung entsprach der im Beispiel 1 (Katalysator, Temperatur, Druck und Versuchsdauer), jedoch wurden andere Ausgangsstoffe eingesetzt.

| Säurederivat | LHSV | GHSV | Umsatz % | Selektivität Aldehyd % | Raum-Zeit-Ausbeute Mol.kg Kat.$^{-1}$.h$^{-1}$ |
|---|---|---|---|---|---|
| 9 Benzoesäuremethylester | 0,11 | 590 | 86 | 82 | 0,620 |
| 10 Benzoylchlorid | 0,10 | 580 | 82 | 45 | 0,320 |

Beispiel 11

Das Beispiel 1 wurde wiederholt, jedoch bei 370°C. Die Versuchsdauer wurde auf 18 h verlängert.

| Carboxylkomponente | LHSV | GHSV | Umsatz % | Selektivität Aldehyd % | Raum-Zeit-Ausbeute Mol.kg Kat.$^{-1}$.h$^{-1}$ |
|---|---|---|---|---|---|
| Benzoesäure | 0,17 | 1000 | 84 | 95 | 1,2 |

Beispiele 12-18

Diese Beispiele wurden bei 370°C an einem $TiO_2$ (92,5 %)/$Cr_2O_3$ (7,5 %)-Katalysator in 4h durchgeführt.

Beispiel 19-21

Diese Beispiele wurden bei 370°C an einem $TiO_2$ (95 %)/CdO (5%)-Katalysator in 4h durchgeführt.

| Bsp. | Carboxyl-Komponente | LHSV | GHSV | Umsatz % | Selektivität Aldehyd % | Raumzeitausbeute Mol·Kg $Kat^{-1}\cdot h^{-1}$ |
|---|---|---|---|---|---|---|
| 12 | p-tert.-Butylbenzoesäure | 0,15 | 700 | 68 | 90 | 0,550 |
| 13 | o-Methylbenzoesäure | 0,13 | 700 | 52 | 90 | 0,480 |
| 14 | Trimethylessigsäure | 0,14 | 700 | 72 | 86 | 0,770 |
| 15 | m-Phenoxybenzoesäure | 0,16 | 700 | 78 | 85 | 0,550 |
| 16 | m-Chlorbenzoesäure | 0,08 | 350 | 70 | 56 | 0,260 |
| 17 | p-Methoxybenzoesäure | 0,17 | 700 | 58 | 84 | 0,630 |
| 18 | Cyclohexancarbonsäure | 0,17 | 700 | 70 | 88 | 0,840 |
| 19 | p-tert.-Butylbenzoesäure | 0,15 | 350 | 97 | 40 | 0,310 |
| 20 | o-Methylbenzoesäure | 0,16 | 350 | 79 | 46 | 0,460 |
| 21 | Trimethylessigsäure | 0,14 | 350 | 84 | 66 | 0,680 |

Beispiel 22

Herstellung eines $VO_2/Cr_2O_3$ Katalysators

y/2 mol Vanadiumpentoxid wurden in 20 %iger wäßriger $H_2SO_4$-Lösung suspensiert. Die Suspension wurde in der Siedehitze mit gasförmigem Schwefeldioxid zum Vanadylsulfat reduziert. Die Vanadylsulfatlösung wurde mit einer Lösung vom x mol eines Chromsalzes in Wasser gemischt und anschließend mit $NH_3$-Lösung bei pH 7-9 gefällt. Die ausgefallenen Hydroxide wurden filtriert und salzfrei gewaschen. Der Rückstand wurde 12 h bei 110°C im Vakuum getrocknet und schließlich 6 h bei 450°C (unter Inertgas) calciniert.

Man erhielt einen Katalysator, der y g-Äquivalente Vanadium und x g-Äquivalente Chrom enthält; für das folgende Beispiel wurden y = 80 und x = 20 eingestellt.

Beispiel 23

Hydrierung von Benzoesäure

| | |
|---|---|
| Katalysator | $VO_2/Cr_2O_3$ (80/20) |
| Substrat | Benzoesäure |
| Temperatur | 380°C |
| Druck | 1,013 bar |
| Versuchsdauer | 30 h |
| Zufuhrrate Säure | 0,15 LHSV ($h^{-1}$) |
| Zufuhrrate Wasserstoff | 800 GHSV ($h^{-1}$) |

| Umsatz | Selek tivität Aldehyd | Selek tivität Toluol | Selek tivität Benzol | Raum-Zeit-Ausbeute |
|---|---|---|---|---|
| 56,5 % | 85 % | 11 | 1,5 | 0,63 mol/kg Kat.h |

**Patentansprüche**

1. Verfahren zur Herstellung von aromatischen und aliphatischen Aldehyden durch katalytische Gasphasenhydrierung der korrespondierenden aromatischen bzw. aliphatischen Carbonsäuren oder ihrer Ester, Anhydride oder Halogenide bei höherer Temperatur mit Wasserstoff, dadurch gekennzeichnet, daß man ein Katalysatorsystem aus Oxiden des Titans und/oder des Vanadiums, wobei mindestens ein Teil des Vanadiums in der Oxidationsstufe IV vorliegt, und eines oder mehrerer Co-Metalle einsetzt, wobei die Co-Metalle aus der Gruppe Chrom, Molybdän, Kobalt, Nickel, Zink, Cadmium und Kupfer ausgewählt werden.

2. Verfahren nach Anspruch 1, dadurch gekennzeichnet, daß das (die) Co-Metall(e) aus der Gruppe Chrom, Molybdän, Nickel, Zink, Cadmium und Kupfer ausgewählt wird (werden) und bevorzugt Chrom ist.

3. Verfahren nach Anspruch 1, dadurch gekennzeichnet, daß das Katalysatorsystem 1 bis 25 g-Äquivalente, bevorzugt 3 bis 12 g-Äquivalente des (der) gewünschten Co-Metall(e), pro 100 g-Äquivalente der Summe aller Metalle enthält.

4. Verfahren nach Anspruch 1, dadurch gekennzeichnet, daß das Katalysatorsystem neben dem Titan bzw. dem Vanadium nur ein Co-Metall enthält.

5. Verfahren nach Anspruch 1, dadurch gekennzeichnet, daß das Titan enthaltende Katalysatorsystem durch folgende Schritte hergestellt wird:
   a) Zugabe von Titantetrachlorid zu einer wäßrigen Lösung von Salz oder Salzen eines oder mehrerer Co-Metalle,
   b) Fällung der Metalle als Hydroxide bei einem pH-Wert von 7 bis 9,

c) Waschen der Hydroxide und anschließende Trocknung,
d) Kalzinieren bei 400 bis 800°C, bevorzugt bei 500 bis 750°C.

6. Verfahren nach Anspruch 1, dadurch gekennzeichnet, daß das Vanadium enthaltende Katalysatorsystem durch folgende Schritte hergestellt wird:
a) Reduktion von Vanadiumpentoxid zu einer wasserlöslichen Vanadylverbindung,
b) Zusammengeben der Vanadylverbindung mit einem oder mehreren wasserlöslichen Salz(en) eines oder mehrerer Co-Metalle,
c) Fällung der Metalle als Hydroxide bei einem pH-Wert von 7-9,
d) Waschen der Hydroxide und anschließende Trocknung,
e) Kalzinieren bei 350 bis 700°C, bevorzugt 400 bis 600°C.

7. Verfahren nach Anspruch 1, dadurch gekennzeichnet, daß aromatische und aliphatische Carbonsäuren und deren Derivate der Formeln

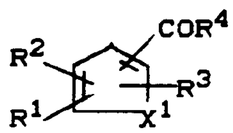

und

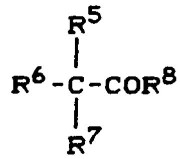

eingesetzt werden, in denen

$R^1$ Wasserstoff, geradkettiges oder verzweigtes $C_1$-$C_8$-Alkyl, geradkettiges oder verzweigtes $C_1$-$C_8$-Alkoxy, $R^3$-substituiertes Phenyl, Naphthyl, $R^3$-substituiertes Phenoxy, $R^3$-substituiertes Benzyl, $R^3$-substituiertes Benzyloxy, Hydroxy, Amino, NH-($C_1$-$C_8$-alkyl), N-($C_1$-$C_8$-alkyl)$_2$, Halogen oder $COR^4$ ist,

$R^2$ für Wasserstoff, geradkettiges oder verzweigtes $C_1$-$C_8$-Alkyl, geradkettiges oder verzweigtes $C_1$-$C_8$-Alkoxy oder $R^3$-substituiertes Phenyl steht,
wobei $R^1$ und $R^2$ gemeinsam einen kondensierten Benzolring bilden können, der mit Hydroxy, Amino, Methyl, Ethyl, Methoxy oder Ethoxy substituiert sein kann,

$R^3$ Wasserstoff, geradkettiges oder verzweigtes $C_1$-$C_8$-Alkyl, geradkettiges oder verzweigtes $C_1$-$C_8$-Alkoxy, Hydroxy, Amino oder Halogen sein kann,

$R^4$ Hydroxy, $C_1$-$C_4$-alkoxy, Chlor, Brom oder die Gruppe

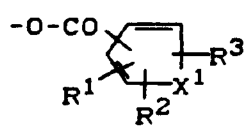

bedeutet,
wobei im letzteren Falle der Anhydridbildung $R^1$ nicht die Bedeutung $COR^4$ annimmt,

$X^1$ für -O-, -N-, -S-, -N=CH- oder -CH=CH- steht,

$R^5$ Wasserstoff oder geradkettiges oder verzweigtes $C_1$-$C_8$-Alkyl bedeutet,

$R^6$ für geradkettiges oder verzweigtes $C_1$-$C_8$-Alkyl, $R^3$-substituiertes Phenyl oder Halogen steht,

$R^7$ für geradkettiges oder verzweigtes $C_1$-$C_8$-Alkyl oder $R^3$-substituiertes Phenyl steht,
wobei das $C_1$-$C_8$-Alkyl durch Halogen, Methoxy oder Ethoxy substituiert sein kann und wobei weiterhin $R^6$ und $R^7$ gemeinsam Dimethylen, Tetramethylen oder Pentamethylen bedeuten, und

$R^8$ Hydroxy, $C_1$-$C_4$-Alkoxy, Chlor, Brom oder die Gruppe -O-CO-C($R^5$,$R^6$,$R^7$) bedeutet.

8. Verfahren nach Anspruch 7, dadurch gekennzeichnet, daß als aromatische Carbonsäuren und deren Derivate

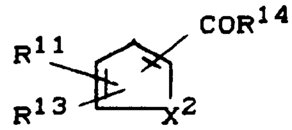

eingesetzt werden, in der

$R^{11}$ Wasserstoff, geradkettiges oder verzweigtes $C_1$-$C_8$-Alkyl, geradkettiges oder verzweigtes $C_1$-$C_8$-Alkoxy, $R^{13}$-substituiertes Phenyl, $R^{13}$-substituiertes Phenoxy, Hydroxy, Amino, NH-($C_1$-$C_8$-alkyl), N-($C_1$-$C_8$-alkyl)$_2$ oder Halogen bedeutet,

$R^{13}$ für Wasserstoff, geradkettiges oder verzweigtes $C_1$-$C_8$-Alkyl, geradkettiges oder verzweigtes $C_1$-$C_8$-Alkoxy, Hydroxy oder Halogen steht,

$R^{14}$ Hydroxy, Methoxy, Ethoxy oder Chlor bedeutet und

$X^2$ für -CH=CH- oder -N=CH-, bevorzugt für -CH=CH- steht, daß als aromatische Carbonsäuren oder deren Derivate bevorzugt solche der Formel

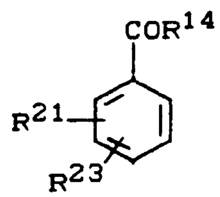

eingesetzt werden, in der

$R^{21}$ Wasserstoff, geradkettiges oder verzweigtes $C_1$-$C_4$-Alkyl, geradkettiges oder verzweigtes $C_1$-$C_4$-Alkoxy, $R^{23}$-substituiertes Phenyl, $R^{23}$-substituiertes Phenoxy, Hydroxy, Amino, Methylamino, Ethylamino, Dimethylamino, Diethylamino, Fluor oder Chlor ist,

$R^{23}$ für Wasserstoff, geradkettiges oder verzweigtes $C_1$-$C_4$-Alkyl, geradkettiges oder verzweigtes $C_1$-$C_4$-Alkoxy, Hydroxy, Fluor oder Chlor steht, und

$R^{14}$ den in Anspruch 7 genannten Bedeutungsumfang hat.

9. Verfahren nach Anspruch 7, dadurch gekennzeichnet, daß als aliphatische Carbonsäuren und deren Derivaten solche der Formel

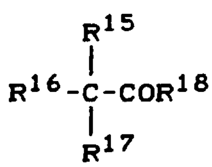

eingesetzt werden, in der

$R^{15}$ für Wasserstoff, Methyl oder Ethyl steht,

$R^{16}$ geradkettiges oder verzweigtes $C_1$-$C_8$-Alkyl oder Phenyl bedeutet,

$R^{17}$ Methyl oder Ethyl bedeutet,
wobei weiterhin $R^{16}$ und $R^{17}$ gemeinsam Tetramethylen oder Pentamethylen bedeuten, und

$R^{18}$ für Hydroxy, Methoxy, Ethoxy oder Chlor steht.

10. Verfahren nach Anspruch 7, dadurch gekennzeichnet, daß als aromatische und aliphatische Carbonsäuren und deren Derivate Benzoesäure, Benzoesäure-methylester, tert.-Butyl-benzoesäure, Phenoxybenzoesäure oder Pivalinsäure eingesetzt werden.

## Claims

1. Process for the preparation of aromatic and aliphatic aldehydes by catalytic gas phase hydrogenation of the corresponding aromatic or aliphatic carboxylic acids or the esters, anhydrides or halides thereof at

elevated temperature using hydrogen, characterized in that a catalyst system composed of oxides of titanium and/or vanadium at least some of the vanadium being in oxidation stage IV, and of one or more co-metals is used, the co-metals being selected from the group consisting of chromium, molybdenum, cobalt, nickel, zinc, cadmium and copper.

**2.** Process according to Claim 1, characterized in that the co-metal(s) is/are selected from the group consisting of chromium, molybdenum, nickel, zinc, cadmium and copper and is preferably chromium.

**3.** Process according to Claim 1, characterized in that the catalyst system contains 1 to 25 g-equivalents, preferably 3 to 12 g-equivalents, of the desired co-metal(s) per 100 g-equivalents of the sum of all metals.

**4.** Process according to Claim 1, characterized in that the catalyst system contains, in addition to the titanium and/or vanadium, only one co-metal.

**5.** Process according to Claim 1, characterized in that the titanium-containing catalyst system is prepared by the following steps:
a) addition of titanium tetrachloride to an aqueous solution of the salt or salts of one or more co-metals,
b) precipitation of the metals as the hgdroxides at a pH of 7 to 9,
c) washing of the hydroxides followed by drying,
d) calcination at 400 to 800°C, preferably at 500 to 750°C.

**6.** Process according to Claim 1, characterized in that the vanadium-containing catalyst system is prepared by the following steps:
a) reduction of vanadium pentoxide to a water-soluble vanadyl compound,
b) combining the vanadyl compound with one or more water-soluble salts of one or more co-metals,
c) precipitation of the metals as the hydroxides at a pH of 7-9,
d) washing of the hydroxides followed by drying,
e) calcination at 350 to 700°C, preferably 400 to 600°C.

**7.** Process according to Claim 1, characterized in that the aromatic and aliphatic carboxylic acids and derivatives thereof which are used have the formulae

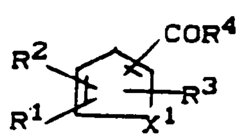

and

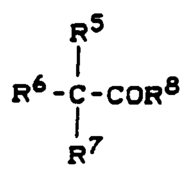

in which

$R^1$ is hydrogen, straight-chain or branched $C_1$-$C_8$-alkyl, straight-chain or branched $C_1$-$C_8$-alkoxy, $R^3$-substituted phenyl, naphthyl, $R^3$-substituted phenoxy, $R^3$-substituted benzyl, $R^3$-substituted benzyloxy, hydroxyl, amino, NH-($C_1$-$C_8$-alkyl), N-($C_1$-$C_8$-alkyl$)_2$, halogen or $COR^4$,

$R^2$ represents hydrogen, straight-chain or branched $C_1$-$C_8$-alkyl, straight-chain or branched $C_1$-$C_8$-alkoxy or $R^3$-substituted phenyl,
where $R^1$ and $R^2$ may together form a condensed benzene ring which may be substituted by hydroxyl, amino, methyl, ethyl, methoxy or ethoxy,

$R^3$ may be hydrogen, straight-chain or branched $C_1$-$C_8$-alkyl, straight-chain or branched $C_1$-$C_8$-alkoxy, hydroxyl, amino or halogen,

$R^4$ is hydroxyl, $C_1$-$C_4$-alkoxy, chlorine, bromine or the group

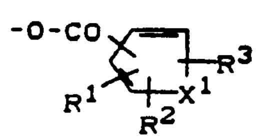

where in the latter case of anhydride formation, $R^1$ does not assume the meaning $COR^4$,

$X^1$ represents -O-, -N-, -S-, -N=CH- or -CH=CH-,

$R^5$ is hydrogen or straight-chain or branched $C_1$-$C_8$-alkyl,
$R^6$ represents straight-chain or branched $C_1$-$C_8$-alkyl, $R^3$-substituted phenyl or halogen,
$R^7$ represents straight-chain or branched $C_1$-$C_8$-alkyl or $R^3$-substituted phenyl,
where the $C_1$-$C_8$-alkyl may be substituted by halogen, methoxy or ethoxy and where furthermore $R^6$ and $R^7$ are together dimethylene, tetramethylene or pentamethylene, and
$R^8$ is hydroxyl, $C_1$-$C_4$-alkoxy, chlorine, bromine or the group -O-CO-C($R^5$,$R^6$,$R^7$).

8. Process according to Claim 7, characterized in that the aromatic carboxylic acids and derivatives thereof which are used are

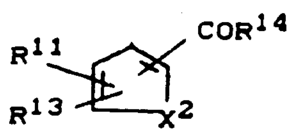

in which
$R^{11}$ is hydrogen, straight-chain or branched $C_1$-$C_8$-alkyl, straight-chain or branched $C_1$-$C_8$-alkoxy, $R^{13}$-substituted phenyl, $R^{13}$-substituted phenoxy, hydroxyl, amino, NH-($C_1$-$C_8$-alkyl), N-($C_1$-$C_8$-alkyl)$_2$ or halogen,
$R^{13}$ represents hydrogen, straight-chain or branched $C_1$-$C_{18}$-alkyl, straight-chain or branched $C_1$-$C_8$-alkoxy, hydroxyl or halogen,
$R^{14}$ is hydroxyl, methoxy, ethoxy or chlorine, and
$X^2$ represents -CH=CH- or -N=CH-, preferably -CH=CH-,
in that the aromatic carboxylic acids or derivatives thereof which are used are preferably those of the formula

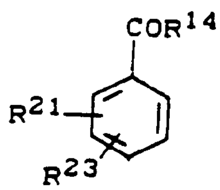

in which
$R^{21}$ is hydrogen, straight-chain or branched $C_1$-$C_4$-alkyl, straight-chain or branched $C_1$-$C_4$-alkoxy, $R^{23}$-substituted phenyl, $R^{23}$-substituted phenoxy, hydroxyl, amino, methylamino, ethylamino, dimethylamino, diethylamino, fluorine or chlorine,
$R^{23}$ represents hydrogen, straight-chain or branched $C_1$-$C_4$-alkyl, straight-chain or branched $C_1$-$C_4$-alkoxy, hydroxyl, fluorine or chlorine, and
$R^{14}$ has the range of meaning given above.

9. Process according to Claim 7, characterized in that the aliphatic carboxylic acids and derivatives thereof which are used are those of the formula

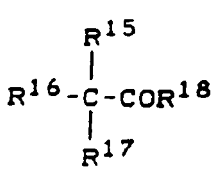

in which
$R^{15}$ represents hydrogen, methyl or ethyl,
$R^{16}$ is a straight-chain or branched $C_1$-$C_8$-alkyl or phenyl,
$R^{17}$ is methyl or ethyl,
$R^{16}$ and $R^{17}$ furthermore together denoting tetramethylene or pentamethylene, and

$R^{18}$ represents hydroxyl, methoxy, ethoxy or chlorine.

**10.** Process according to Claim 7, characterized in that the aromatic and aliphatic carboxylic acids and derivatives thereof which are used are benzoic acid, methyl benzoate, tert-butylbenzoic acid, phenoxybenzoic acid or pivalic acid.

## Revendications

**1.** Procédé de production d'aldéhydes aromatiques et aliphatiques par hydrogénation catalytique en phase gazeuse des acides carboxyliques aromatiques et respectivement aliphatiques correspondants ou de leurs esters, leurs anhydrides ou leurs halogénures à température élevée avec de l'hydrogène, caractérisé en ce qu'on utilise une composition de catalyseur constituée d'oxydes de titane et/ou de vanadium, une partie au moins du vanadium étant au degré d'oxydation IV, et d'un ou plusieurs métaux d'accompagnement, les métaux d'accompagnement étant choisis dans le groupe comprenant le chrome, le molybdène, le cobalt, le nickel, le zinc, le cadmium et le cuivre.

**2.** Procédé suivant la revendication 1, caractérisé en ce que le ou les métaux d'accompagnement sont choisis dans le groupe comprenant le chrome, le molybdène, le nickel, le zinc, le cadmium et le cuivre, notamment le chrome.

**3.** Procédé suivant la revendication 1, caractérisé en ce que la composition de catalyseur contient 1 à 25 équivalents-grammes, de préférence 3 à 12 équivalents-grammes du ou des métaux désirés d'accompagnement pour 100 équivalents-grammes de la somme de tous les métaux.

**4.** Procédé suivant la revendication 1, caractérisé en ce que la composition de catalyseur contient un seul métal d'accompagnement à côté du titane ou du vanadium.

**5.** Procédé suivant la revendication 1, caractérisé en ce que la composition de catalyseur contenant du titane est préparée par les étapes suivantes :
a) addition de tétrachlorure de titane à une solution aqueuse d'un ou plusieurs sels d'un ou plusieurs métaux d'accompagnement,
c) précipitation des métaux sous la forme d'hydroxydes à une valeur de pH de 7 à 9,
c) lavage des hydroxydes suivi d'un séchage,
d) calcination à 400-800°C, de préférence de 500-750°C.

**6.** Procédé suivant la revendication 1, caractérisé en ce que la composition de catalyseur contenant du vanadium et préparée par les étapes suivantes :
a) réduction du pentoxyde de vanadium en un composé de vanadyle soluble dans l'eau,
b) réunion du composé de vanadyle avec un ou plusieurs sels hydrosolubles d'un ou plusieurs métaux d'accompagnement,
c) précipitation des métaux sous forme d'hydroxydes à une valeur de pH de 7 à 9,
d) lavage des hydroxydes suivi d'un séchage,
e) calcination à 350-700°C, de préférence à 400-600°C.

**7.** Procédé suivant la revendication 1, caractérisé en ce qu'on utilise des acides carboxyliques aromatiques et aliphatiques et leurs dérivés de formules

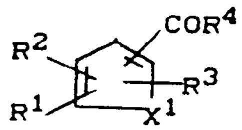

et

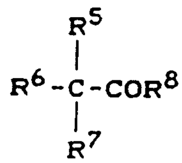

dans lesquelles

$R^1$ représente l'hydrogène, un groupe alkyle en $C_1$ à $C_8$ linéaire ou ramifié, un groupe alkoxy en $C_1$ à $C_8$ linéaire ou ramifié, un groupe phényle à substituant $R^3$, un groupe naphtyle, un groupe phénoxy à substituant $R^3$, benzyle à substituant $R^3$, benzyloxy à substituant $R^3$, hydroxy, amino, NH-

(alkyle en $C_1$ à $C_8$), N-(alkyle en $C_1$ à $C_8$)$_2$, un halogène ou un groupe $COR^4$,

$R^2$ représente l'hydrogène, un groupe alkyle en $C_1$ à $C_8$ linéaire ou ramifié, alkoxy en $C_1$ à $C_8$ linéaire ou ramifié ou phényle à substituant $R^3$, $R^1$ et $R^2$ pouvant former ensemble un noyau benzénique condensé qui peut être substitué avec un radical hydroxy, amino, méthyle, éthyle, méthoxy ou éthoxy,

$R^3$ peut être de l'hydrogène, un radical alkyle en $C_1$ à $C_8$ linéaire ou ramifié, alkoxy en $C_1$ à $C_8$ linéaire ou ramifié, hydroxy, amino ou un halogène,

$R^4$ désigne un groupe hydroxy, alkoxy en $C_1$ à $C_4$, du chlore, du brome ou le groupe

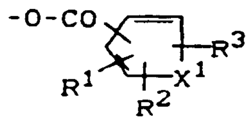

$R^1$ ne recevant pas la définition $COR^4$ dans le dernier cas de formation d'un anhydride,

$X^1$ représente -O-, -N-, -S-, -N=CH- ou -CH=CH-,

$R^5$ représente l'hydrogène ou un groupe alkyle en $C_1$ à $C_8$ linéaire ou ramifié,

$R^6$ est un groupe alkyle en $C_1$ à $C_8$ linéaire ou ramifié, phényle à substituant $R^3$ ou un halogène,

$R^7$ est un groupe alkyle en $C_1$ à $C_8$ linéaire ou ramifié ou phényle à substituant $R^3$, le groupe alkyle en $C_1$ à $C_8$ pouvant être substitué par un halogène, un radical méthoxy ou éthoxy et en outre, $R^6$ et $R^7$ désignant ensemble un groupe diméthylène, tétraméthylène ou pentaméthylène,

et

$R^8$ est un groupe hydroxy, alkoxy en $C_1$ à $C_4$ , du chlore, du brome ou le groupe -O-CO-C($R^5$,$R^6$,$R^7$).

**8.** Procédé suivant la revendication 7, caractérisé en ce qu'on utilise des acides carboxyliques aromatiques et leurs dérivés de formule

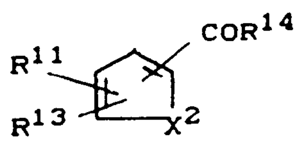

dans laquelle

$R^{11}$ représente l'hydrogène, un groupe alkyle en $C_1$ à $C_8$ linéaire ou ramifié, un groupe alkoxy en $C_1$ à $C_8$ linéaire ou ramifié, un groupe phényle à substituant $R^{13}$, phénoxy à substituant $R^{13}$, hydroxy, amino, NH-(alkyle en $C_1$ à $C_8$), N-(alkyle en $C_1$ à $C_8$)$_2$ ou un halogène,

$R^{13}$ représente l'hydrogène, un groupe alkyle en $C_1$ à $C_8$ linéaire ou ramifié, alkoxy en $C_1$ à $C_8$ linéaire ou ramifié, hydroxy ou un halogène,

$R^{14}$ est un groupe hydroxy, méthoxy, éthoxy ou du chlore et

$X^2$ représente -CH=CH- ou -N=CH-, de préférence -CH=CH-.

et en ce qu'on utilise les acides carboxyliques aromatiques ou leurs dérivés de formule

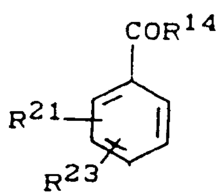

dans laquelle

$R^{21}$ représente l'hydrogène, un groupe alkyle en $C_1$ à $C_4$ linéaire ou ramifié, un groupe alkoxy en $C_1$ à $C_4$ linéaire ou ramifié, un groupe phényle à substituant $R^{23}$, phénoxy à substituant $R^{23}$, hydroxy, amino, méthylamino, éthylamino, diméthylamino, diéthylamino, du fluor ou du chlore,

$R^{23}$ représente l'hydrogène, un groupe alkyle en $C_1$ à $C_4$ linéaire ou ramifié, alkoxy en $C_1$ à $C_4$ linéaire

ou ramifié, un groupe hydroxy, du fluor ou du chlore et

$R^{14}$ a les limites de définition mentionnées dans la rewendication 7.

**9.** Procédé suivant la revendication 7, caractérisé en ce qu'on utilise des acides carboxyliques aliphatiques et leurs dérivés qui répondent à la formule

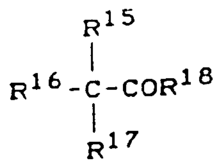

dans laquelle

$R^{15}$ représente l'hydrogène, un groupe méthyle ou éthyle,

$R^{16}$ est un groupe méthyle en $C_1$ à $C_8$ linéaire ou ramifié ou phényle,

$R^{17}$ est un groupe méthyle ou éthyle,

en outre, $R^{16}$ et $R^{17}$ désignant ensemble un groupe tétraméthylène ou pentaméthylène et

$R^{18}$ est un groupe hydroxy, méthoxy, éthoxy ou du chlore.

**10.** Procédé suivant la revendication 7, caractérisé en ce qu'on utilise comme acides carboxyliques aromatiques et aliphatiques et leurs dérivés, l'acide benzoïque, l'ester méthylique d'acide benzoïque, l'acide tertio-butyl-benzoïque, l'acide phénoxybenzoïque ou l'acide pivalique.